# EUROPEAN PATENT APPLICATION

(11) **EP 4 609 825 A1**
(43) Date of publication of application: **03.09.2025**
(21) Application number: 24159999.2
(22) Date of filing: 27.02.2024
(51) Int. Cl.: A61C 19/04, A61C 8/00

(54) **PERIODONTAL PROBE WITH RESTRICTED FLEXIBILITY FOR PERI-IMPLANT PROBING**

(71) Applicant: Brum Oral Surgery Ltd., Birmingham B29 7QN (GB)
(72) Inventor: DIETRICH, Thomas, Birmingham B29 7QN (GB)
(74) Representative: Bardehle Pagenberg Partnerschaft mbB Patentanwälte Rechtsanwälte

(57) **Abstract**

The present disclosure relates to a periodontal probe configured for safe, precise, and reliable peri-implant probing. A periodontal probe disclosed herein comprises a handle, and a probe tip comprising a proximal end connected to the handle and a flexible distal end for probing an interface between a dental implant and a gingival tissue / peri-implant mucosa surrounding the dental implant. The flexible distal end is configured such that a flexibility of the flexible distal end is at least 30% larger, preferably at least 50% larger, more preferably at least 100% larger, and even more preferably at least 200% larger in a bending direction of the probe tip than in a non-bending direction of the probe tip.

## Description

### Technical field

The present invention relates to a periodontal probe, in particular a peri-implant probe comprising a flexible tip end with restricted flexibility that facilitates safe and reliable probing of gingival tissues surrounding dental implants.

### Technical background

Periodontal probes are used routinely in clinical dental practice to establish the periodontal probing depth around teeth, which is required for every comprehensive dental examination. These probes are therefore standard equipment in every dental practice. Probing around dental implants (peri-implant probing) is also recommended to monitor the condition of peri-implant soft and hard tissues. However, with standard periodontal probes probing around dental implants can be challenging and is often impossible, due to the straight probe not being compatible with the curved emergence profile of implant restorations.

### Summary

A typical problem encountered with standard periodontal probes is their rigidity, which makes them unsuitable for peri-implant probing in many clinical situations. The problem is the difference in the emergence profiles between root / crown and implant/crown complexes, as implants have circular and very often smaller diameters at the marginal bone level compared to natural teeth. This often results in significantly curved emergence profiles of implant restorations, which cannot be probed with conventional straight periodontal probes.

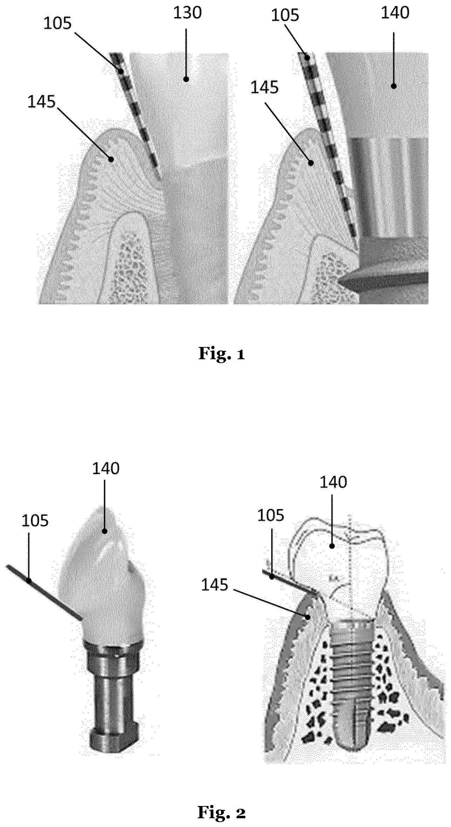

**Fig. 1** illustrates probing an interface between gingival tissue 145 and a natural tooth 130 (left panel) as well as an interface between gingival tissue 145 and a dental implant 140 (right panel) using a conventional periodontal probe comprising a slightly tapered and rigid tip end 105. In this example, the emergence profile of the implant / crown complex 140 is similar to that of a natural tooth 130 and there is no problem using the rigid standard periodontal probe tip for probing the gingival pocket at the interface between gingival tissue 145 and a dental implant 140.

**Fig. 2** illustrates the situation mentioned above where the implant / crown complex 140 exhibits a curved emergence profile preventing peri-implant probing with a standard periodontal probe that typically have a slightly tapered and rigid probe tip 105 with a circular cross section (see Fig. 1). In particular, as shown in the right panel of Fig. 2 it is not possible to reliably determine the depth of a gingival pocket at the interface between the implant 140 and the gingival tissue 145 surrounding it. As known to the person skilled in the art, the interface between a dental implant and the surrounding tissue may also be designated as interface between a dental implant and a peri-implant mucosa surrounding the dental implant. Here, and for the present disclosure the terms mucosa and gingiva are thus used interchangeably.

To address such and similar shortcomings of conventional periodontal probes, the present disclosure provides a periodontal probe, configured for peri-implant probing, comprising a handle, and a probe tip comprising a proximal end connected to the handle and a flexible distal end for probing an interface between a tooth or a dental implant and a gingival tissue surrounding the tooth or the dental implant. The flexible distal end is configured such that a flexibility of the flexible distal end is at least 30% larger, preferably at least 50% larger, more preferably at least 100% larger, and even more preferably at least 200% larger in a bending direction of the probe tip than in a non-bending direction of the probe tip. Typically, in use, the bending direction may essentially point radially outwards from the tooth or dental implant and the non-bending direction may be essentially tangential to a circle around the centra line of the tooth or dental implant.

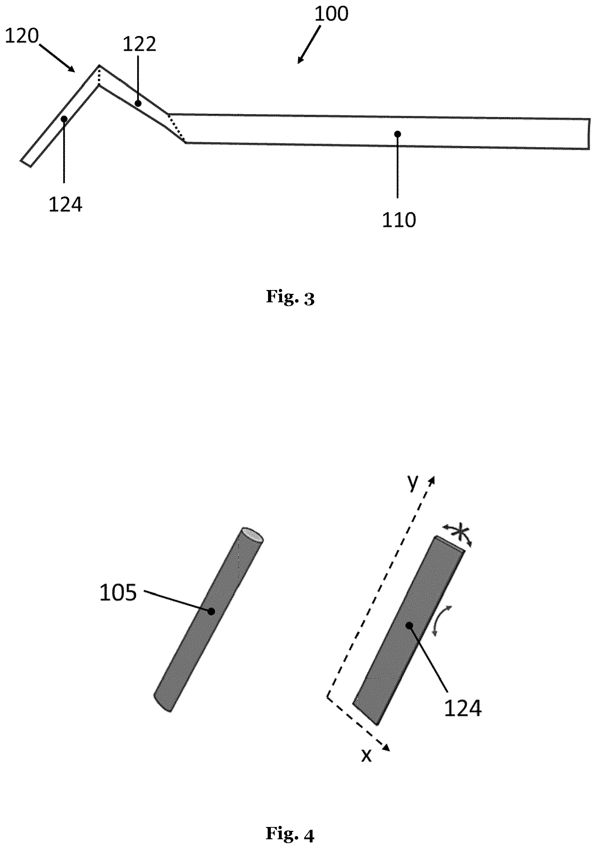

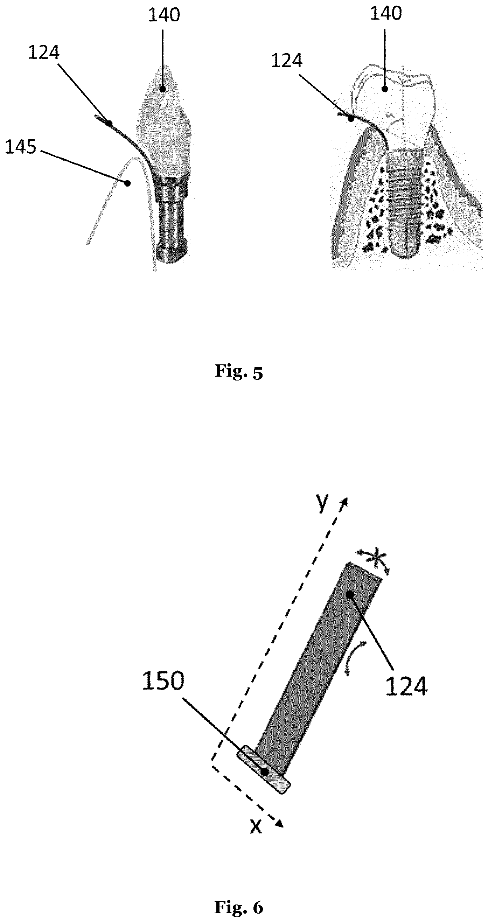

For example, in some implementations, the probe tip may be configured such that a force needed for bending the flexible distal end by at least 5% in the bending direction is at least 30% smaller, preferably at least 50% smaller, more preferably at least 100% smaller, and even more preferably at least 200% smaller than a force needed to bend the flexible distal end by at least 5% in the non-bending direction. Further, in some implementations, the flexible distal end of the probe tip may comprise a cross-sectional shape, preferably an elliptic, trapezoidal, or rectangular shape, that has an aspect ratio of at least 1.3, preferably of at least 1.5 and more preferably of at least 2.0. (see **Fig. 4** and **Fig. 6****).** In this manner, it can be ensured that the flexibility of the probe tip is restricted in the non-bending direction, while being used for precise and reliable peri-implant probing in the bending direction (see **Fig. 5****)**

Alternatively, or additionally, in some implementations, the flexible distal end may comprise an asymmetric flexible material, wherein an intrinsic bending modulus of the asymmetric flexible material may comprise an asymmetry of at least 30%, preferably of at least 50 %, more preferably of at least 100%, and even more preferably at least 200%.

In some specific implementations, the flexible distal end of the probe tip may comprise a cross-sectional shape that has an aspect ratio of at least 4.0, preferably of at least 8.0. For example, the flexible distal may have a cuboid shape (tapered or not) that may have a rectangular cross-section, wherein the long side of the rectangle may be 4.0 or 8.0 times larger than the short side of the rectangle. For example, the short side may be 0,5 mm and the long side may be 2.0mm or 4.0mm. Similar dimensions, compatible with human anatomy, are also part of the present disclosure.

The present disclosure also relates to a periodontal probe system for peri-implant probing comprising: a probe handle configured to be releasably connected to a plurality of periodontal probe tips, and a plurality of periodontal probe tips as specified above and described below, wherein the probe tips are configured to be releasably connected to the probe handle, and wherein each periodontal probe tip of a subset of the plurality of periodontal probe tips comprises a different ratio of flexibility of the flexible distal end in the bending direction as compared to the non-bending direction. For example, such a system may comprise probe tips having a different aspect ratio, a different material, and / or a different cross-sectional shape.

The present disclosure also relates to a method for measuring a depth of a periodontal pocket in a gingiva adjacent a tooth or dental implant, said method comprising inserting a periodontal probe as specified above and described below into the periodontal pocket until a most distal end of the flexible distal end of the probe tip reaches a bottom of the periodontal pocket, and determining a depth of the periodontal pocket, wherein inserting the periodontal probe into the periodontal pocket comprises bending the flexible distal end of the probe tip in the bending direction by more than 5%, preferably by more than 30%, while substantially not bending the flexible distal end of the probe tip in the non-bending direction. Further aspects of the present disclosure, implementation details, and some related benefits are described in the following with reference to the appended drawings.

### Brief description of the drawings

**Fig. 1** is a schematic illustration showing use of a conventional periodontal probe for probing an interface between a tooth or dental implant and the surrounding gingival tissue;
**Fig. 2** is a schematic illustration showing use of a conventional periodontal probe for probing an interface between a dental implant with curved emergence profile and the surrounding gingival tissue;
**Fig. 3** is a schematic illustration showing a periodontal probe according to aspects of the present disclosure;
**Fig. 4** is a schematic illustration showing a part of a conventional probe tip (left panel) and a part of a probe tip with restricted flexibility according to aspects of the present disclosure (right panel);
**Fig. 5** is a schematic illustration showing use of a conventional periodontal probe for probing an interface between a dental implant with curved emergence profile and the surrounding gingival tissue.
**Fig. 6** is a schematic illustration showing a part of a probe tip with restricted flexibility and a tissue protection element according to aspects of the present disclosure;
**Fig. 7** is a schematic illustration showing a further periodontal probe according to aspects of the present disclosure;
**Fig. 8** is a schematic process flow diagram of a method for measuring a depth of a periodontal pocket according to aspects of the present disclosure.

### Detailed description of illustrative examples

In the following, some exemplary embodiments of the present disclosure are described in more detail, with reference to exemplary periodontal probes, systems and methods for peri-implant probing. While specific feature combinations are described in the following paragraphs with respect to the exemplary embodiments of the present disclosure, it is to be understood that not all features of the discussed embodiments have to be present for realizing the disclosure, which is defined by the subject matter of the claims. The disclosed embodiments may be modified by combining certain features of one embodiment with one or more technically and functionally compatible features of other embodiments. Specifically, the skilled person will understand that features, components, procedural steps and / or functional elements of one embodiment can be combined with technically compatible features, procedural steps, components and / or functional elements of any other embodiment of the present disclosure if covered by the subject matter as specified by the appended claims.

Specifically, as schematically illustrated in **Fig. 3** and **Fig. 4** aspects of the present disclosure relate to a periodontal probe 100, comprising a handle 110, and a probe tip 120 comprising a proximal end 122 connected to the handle 110 and a flexible distal end 124 for probing an interface between a tooth 130 or a dental implant 140 and a gingival tissue 145 surrounding the tooth 130 or the dental implant 140. For example, the flexible distal end 124 may be used for probing a gingival crevice, sulcus, pocket or similar anatomical structure. The flexible distal end 124 of the probe tip 120 is configured such that it comprises a restricted flexibility. For example, the flexible distal end 124 may be flexible in one direction and essentially rigid in another direction. For example, a flexibility of the flexible distal end 124 may be at least 30% larger, preferably at least 50% larger, more preferably at least 100% larger, and even more preferably at least 200% larger in a bending direction of the probe tip 120 than in a non-bending direction of the probe tip 120. In this manner, aspects of the present disclosure allow to precisely and reliably probe the interface between dental implants 140 that may comprise a significantly curved emergence profile and the surrounding gingival tissue 145 without causing pain and / or tissue damage to the patient.

As mentioned above, in some implementation of the present disclosure, the probe tip 120 may be configures such that a force needed for bending the flexible distal end 124 by at least 5% in the bending direction is at least 30% smaller, preferably at least 50% smaller, more preferably at least 100% smaller, and even more preferably at least 200% smaller than a force needed to bend the flexible distal end 124 by at least 5% in the non-bending direction. For example, as illustrated in **Fig. 4****,** the flexible distal end 124 of the probe tip 120 may comprise a cross-sectional shape, preferably an elliptic, trapezoidal, or rectangular shape, that has an aspect ratio of at least 1.3, preferably of at least 1.5 and more preferably of at least 2.0. In some cases, the flexible distal end 124 may even comprise a cross-sectional shape that has an aspect ratio of at least 4.0, preferably of at least 8.0. For example, as schematically illustrated in **Fig. 4****,** right panel, the flexible distal end 124 may have a width X of 4 mm or 5 mm and a thickness Y of 0.5 mm. Of course, other anatomically suitable dimensions are also part of the present disclosure.

As schematically illustrated in **Fig. 6****,** in some implementations, the flexible distal end 124 of the probe tip 120 may comprise a tissue protection element 150 that may be arranged at a most distal end of the flexible distal end 124 of the probe tip 120, and that may comprise a three-dimensional shape, preferably an ellipsoid shape, that has a larger cross-section than a cross-section of a part of the flexible distal end 124 that is directly connected to the tissue protection element. In this manner, a likelihood of causing pain or tissue damage when using the probes disclosed herein can further be reduced.

Alternatively or additionally, the flexible distal end 124 of the probe tip 120 may also comprise an asymmetric flexible material, wherein an intrinsic bending modulus of the asymmetric flexible material may comprise an asymmetry of at least 30%, preferably of at least 50 %, more preferably of at least 100%, and even more preferably at least 200%. Further, the flexible distal end 124 or the probe tip 120 may also comprises one or more of: a super-elastic material, preferably nickel titanium, NiTi, and a shape-memory material, preferably a shape-memory alloy. For example, materials used for modern root canal probes may also be used. Using such a shape-memory material facilities, e.g., that the probe resets after bending, i.e. the probe adapts to the emergence profile of the implant during probing and then resets again.

In some implementations, the flexible distal end 124 may comprise probe tip insertion distance indications, preferably using a mm scale. Further, a connection of the probe tip 120 to the handle 110 of the periodontal probe 100 may be configured to be releasable, such that different probe tips 120 with different bending properties and / or shapes can be connected to a single handle 110. Further, as mentioned above, in use, the bending direction typically points essentially radially outwards from the tooth or implant. Similarly, the non-bending direction typically points essentially in the direction of the interface between the tooth 130 or the dental implant 140 and the gingival tissue 145 surrounding the tooth 130 or implant 140.

In some implementations, the periodontal probe 100 may also comprise a first, and a second probe tip 120 connected to opposite ends of the handle 110, wherein the first probe tip 120 may be different from the second probe tip 120. For example, a flexibility of the flexible distal end 124 of the first probe tip 120 may be different from a flexibility of the flexible distal end 124 of the second probe tip 120. Further, an aspect ratio of the flexible distal end 124 of the first probe tip 120 may be different from a flexibility of the flexible distal end 124 of the second probe tip 120. Further, also an asymmetric flexible material of the flexible distal end 124 of the first probe tip 120 may be different from an asymmetric flexible material of the flexible distal end 124 of the second probe tip 120.

Further, also an orientation of the flexible distal end of the first probe tip 120 with respect to a longitudinal cross-sectional plane of the handle 100 or the first probe tip may differ from an orientation of the flexible distal end 124 of the second probe tip 120 with respect to the longitudinal cross-sectional plane of the handle 110 or the second probe tip. For instance, the flexible distal end 124 of the first probe tip 120 may be oriented with respect to the longitudinal cross-sectional plane of the handle 110 by an angle λ ≥ 30°, preferably λ = 45°, and the flexible distal end 124 of the second probe tip may be oriented with respect to the longitudinal cross-sectional plane of the handle by an angle λ ≤ - 30°, preferably λ =-45°. For example, such a probe may be used to probe multiple dental implants at different location within the jaw of a patient without having to change the instrument.

Further, the probes disclosed herein may also be used in a periodontal probe system comprising a probe handle 110 configured to be releasably connected to a plurality of periodontal probe tips 120 as well as such a plurality of periodontal probe tips 120 as specified herein that are configured to be releasably connected to the probe handle 110. For example, each periodontal probe tip 120 of the plurality of periodontal probe tips may comprise a different ratio of flexibility of the flexible distal end in the bending direction as compared to the non-bending direction.

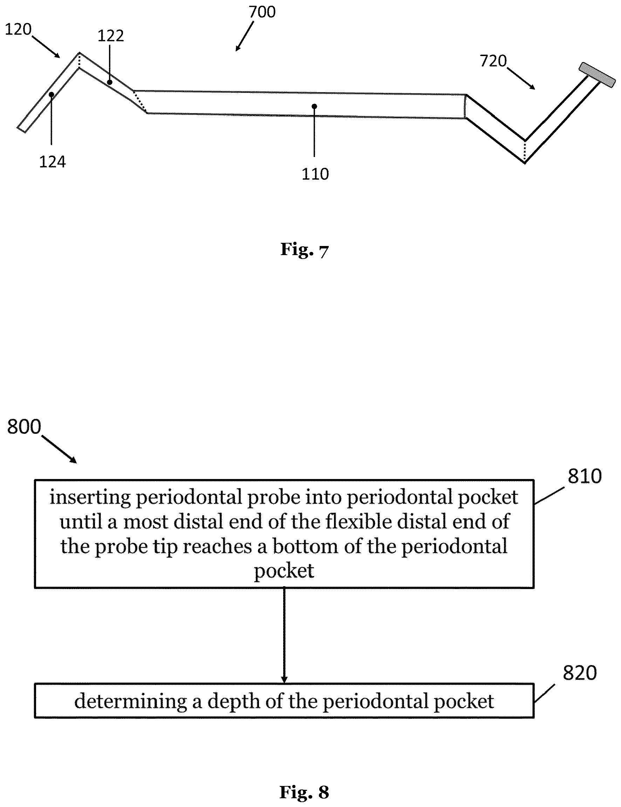

**Fig. 7** illustrates a further exemplary periodontal probe 700 according to aspects of the present disclosure. The periodontal probe 700 is similar to the periodontal probe 100 discussed above and includes a second probe tip 720 that may exhibit on ore more different probing properties as compared to the first probe tip 120 as discussed above.

**Fig. 8** illustrates a method for measuring a depth of a periodontal pocket in a gingiva 145 adjacent a tooth 130 or dental implant 140, said method comprising inserting 810 a periodontal probe 100, 700 as specified herein into the periodontal pocket until a most distal end of the flexible distal end 124 of the probe tip 120 reaches a bottom of the periodontal pocket, and determining 820 a depth of the periodontal pocket, wherein inserting 810 the periodontal probe 100, 700 into the periodontal pocket comprises bending 715 the flexible distal end 124 of the probe tip 120 in the bending direction by more than 5%, preferably by more than 30%, while substantially not bending the flexible distal end 124 of the probe tip 120 in the non-bending direction.

While the present specification contains many specific implementation details, these should not be construed as limitations on the scope of any invention or of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination. Similarly, while operations are depicted in the drawings in a particular order, this should not be understood as requiring that such operations be performed in the particular order shown or in sequential order, or that all illustrated operations be performed, to achieve desirable results.

## Claims

1. Periodontal probe (100), comprising:
a handle (110); and
a probe tip (120) comprising a proximal end (122) connected to the handle and a flexible distal end (124) for probing an interface between a dental implant (140) and a gingival tissue (145) surrounding the dental implant (140),
wherein the flexible distal end is configured such that a flexibility of the flexible distal end is at least 30% larger, preferably at least 50% larger, more preferably at least 100% larger, and even more preferably at least 200% larger in a bending direction of the probe tip than in a non-bending direction of the probe tip.

2. Periodontal probe of claim 1,
wherein a force needed for bending the flexible distal end by at least 5% in the bending direction is at least 30% smaller, preferably at least 50% smaller, more preferably at least 100% smaller, and even more preferably at least 200% smaller than a force needed to bend the flexible distal end by at least 5% in the non-bending direction.

3. Periodontal probe of claim 1 or claim 2,
wherein the flexible distal end comprises a cross-sectional shape, preferably an elliptic, trapezoidal, or rectangular shape, that has an aspect ratio of at least 1.3, preferably of at least 1.5 and more preferably of at least 2.0.

4. Periodontal probe of any of claims 1 to 3, wherein the flexible distal end comprises an asymmetric flexible material, wherein an intrinsic bending modulus of the asymmetric flexible material comprises an asymmetry of at least 30%, preferably of at least 50 %, more preferably of at least 100%, and even more preferably at least 200%.

5. Periodontal probe of any of the preceding claims 1 to 4, wherein
the flexible distal end comprises a cross-sectional shape that has an aspect ratio of at least 4.0, preferably of at least 8.0.

6. Periodontal probe of any of claims 1 to 5, wherein the flexible distal end comprises one or more of: a super-elastic material, preferably nickel titanium, NiTi, and a shape-memory material, preferably a shape-memory alloy.

7. Periodontal probe of any of the preceding claims 1 to 6, wherein the flexible distal end comprises probe tip insertion distance indications, preferably using a mm scale.

8. Periodontal probe of any of claims 1 to 7, wherein the flexible distal end comprises a tissue protection element that is arranged at a most distal end of the flexible distal end of the probe tip, and that comprises a three-dimensional shape, preferably an ellipsoid shape, that has a larger cross-section than a cross-section of a part of the flexible distal end that is directly connected to the tissue protection element.

9. Periodontal probe of any of claims 1 to 8, wherein a connection of the probe tip to the handle of the periodontal probe is releasable, such that different probe tips with different bending properties and / or shapes can be connected to a single handle.

10. Periodontal probe of any of claims 1 to 9,
wherein, in use, the bending direction points essentially radially outwards from
the implant; and / or
wherein, in use, the non-bending direction points essentially in the direction of the interface between the interface between the dental implant and the gingival tissue surrounding the implant.

11. Periodontal probe of any of claims 1 to 10,
comprising a first and a second probe tip connected to opposite ends of the handle, wherein the first probe tip is different from the second probe tip; and
wherein a flexibility of the flexible distal end of the first probe tip is different from a flexibility of the flexible distal end of the second probe tip; and / or
wherein an aspect ratio of the flexible distal end of the first probe tip is different from a flexibility of the flexible distal end of the second probe tip; and / or
wherein an asymmetric flexible material of the flexible distal end of the first probe tip is different from an asymmetric flexible material of the flexible distal end of the second probe tip; and / or
wherein an orientation of the flexible distal end of the first probe tip with respect to a longitudinal cross-sectional plane of the handle or the first probe tip differs from an orientation of the flexible distal end of the second probe tip with respect to the longitudinal cross-sectional plane of the handle or the second probe tip.

12. Periodontal probe of claim 11, wherein the flexible distal end of the first probe tip is oriented with respect to the longitudinal cross-sectional plane of the handle by an angle λ ≥ 30°, preferably λ = 45°, and wherein the flexible distal end of the second probe tip is oriented with respect to the longitudinal cross-sectional plane of the handle by an angle λ ≤ - 30°, preferably λ = - 45°.

13. A periodontal probe system comprising:
a probe handle configured to be releasably connected to a plurality of periodontal probe tips;
and a plurality of periodontal probe tips as specified in any of the preceding claims 1 to 12, and configured to be releasably connected to the probe handle;
wherein each periodontal probe tip of a subset of the plurality of periodontal probe tips comprises a different ratio of flexibility of the flexible distal end in the bending direction as compared to the non-bending direction.

14. A method for measuring a depth of a periodontal pocket in a gingiva adjacent a dental implant, said method comprising:
inserting the periodontal probe of any of the preceding claims 1 to 13 into the periodontal pocket until a most distal end of the flexible distal end of the probe tip reaches a bottom of the periodontal pocket; and
determining a depth of the periodontal pocket; wherein inserting the periodontal probe into the periodontal pocket comprises:
bending the flexible distal end of the probe tip in the bending direction by more than 5%, preferably by more than 30%, while substantially not bending the flexible distal end of the probe tip in the non-bending direction.
